# EUROPEAN PATENT SPECIFICATION

(11) **EP 1 765 259 B1**
(45) Date of publication and mention of the grant of the patent: **09.02.2011**
(21) Application number: 05769564.5
(22) Date of filing: 01.07.2005
(51) Int. Cl.: A61K 8/37, A61K 8/31, A61K 8/81, A61Q 19/00

(54) **COSMETIC COMPOSITIONS AND METHODS FOR SENSORY COOLING**
KOSMETISCHE ZUSAMMENSETZUNGEN UND VERFAHREN MIT KÜHLENDER WIRKUNG
COMPOSITIONS COSMETIQUES ET PROCEDES DESTINES A PROVOQUER UNE SENSATION RAFRAICHISSANTE

(30) Priority: 01.07.2004 US 584568 P
(43) Date of publication of application: 28.03.2007
(73) Proprietor: E-L Management Corp., New York, NY 10153 (US)
(72) Inventor: MOHAMMADI, Fatemeh, Hauppauge, New York 11788 (US); HARRISON, James, Blue Point, New York 11715 (US); ALUSKEWICZ, Laurie, Commack, New York 11725 (US)
(74) Representative: Hirsch & Associés
(86) International application number: PCT/US2005/023534
(87) International publication number: WO 2006/007564

(56) References cited:
- WO-A-93/25177
- WO-A-03/007908
- FR-A- 2 825 631
- US-A- 5 474 979
- US-A- 5 914 117
- KEN KLEIN: "FORMULATING WATER-RESISTANT SUNSCREEN EMULSIONS" COSMETICS & TOILETRIES, WHEATON, IL, US, February 2002 (2002-02), pages 1-3, XP008079745 ISSN: 0361-4387

## Description

### Field of the Invention

The present invention relates to skin care cosmetic compositions and methods. In particular, the present invention relates to novel cosmetic compositions and methods for causing a sensory cooling sensation.

### Background of the Invention

A common practice in the art involves compositions containing components that cause a cooling sensation to the skin. Such compositions include perfumes, lotions, shaving creams and gels, post-shaving preparations, shampoos, antiperspirants, deodorants, anti-acne medicines, first aid ointments, and a variety of other skin care and pharmaceutical products that are applied to the skin.

Menthol is often used to provide such a cooling sensation on the skin. Known as a physiological coolant, menthol is believed to act as a direct stimulus on the cold receptors at the nerve endings which in turn stimulate the central nervous system. However, menthol is limited in use because of its strong odor and its relative volatility. The high volatility of menthol limits the period of time which it can provide a cooling sensation and can also result in a stinging sensation in the eye if applied in near proximity to the eye. The cooling effect of menthol and other related terpene alcohols and their derivatives has also been reported in Koryo, 95, (1970), pp. 39-43. A specific menthol, 2, 3-p-menthane diol has also been reported as having a sharp cooling taste (Beilstein, Handbuch der Organischen Cheme, 4th Ed. (1923) Vol. 6, p. 744).

Various additives such as ethanols and volatile alcohols have been used in compositions to cause cooling effects. However, because of the volatility of the alcohols, the cooling sensation is short lived. Moreover, the volatile alcohols tend to impart a stinging sensation along with the cooling sensation.

Therefore, there remains a need for compositions and methods for improved cooling effect that overcome the stinging and volatility problems of the known prior art.

### Summary of the Invention

The present invention comprises a composition for topical application to the skin according to claim 1.

The present invention further comprises methods for causing a cooling sensation on the skin according to claim 1.

### Detailed Description

Except in operating and comparative examples, or where otherwise explicitly indicated, all numbers in this description indicating amounts or ratios of material or conditions of reaction, physical properties of materials and/or use are to be understood as modified by the word "about." All amounts are by weight of the final composition, unless otherwise specified.

The following includes a list of definitions for terms used in the application.

By "cooling sensation" is meant a sensation causing the skin temperature to drop for at least 10 seconds, as measured by the heat of fusion of the composition.

By "skin temperature" is meant the temperature on the top layer of human or mammalian skin at a temperature of between 35 and 40°C.

By "unsolubilized" is meant the component remains substantially in its crystalline form when incorporated within a composition.

By "substantially unsolubilized" is meant at least 85% of the component remains unsolubilized.

By "effective amount" is meant an amount of an agent (e.g., cooling ingredient) high enough to provide a cooling sensation on the body temperature within the scope of sound sensory judgment.

While not wishing to be bound by any theories, it is believed that a cooling sensation can be caused when highly volatile substances contact the skin. The cooling sensation occurs as the substance evaporates off the skin, thereby cooling the external skin temperature. A cooling sensation can also be caused when the skin is contacted by components that absorb heat from the skin, thereby causing a drop in the external skin temperature. A cooling sensation caused by absorption of heat is believed to have a longer effect.

Ingredients are believed to absorb heat from the skin to facilitate melting when applied to the skin. The heat of fusion is the heat absorbed by a unit of mass of a solid chemical element at its melting point in order to convert the solid into a liquid at the same temperature. The energy that goes into melting a solid is used to dissociate the intermolecular bonds holding its molecules in place rather than to increase the average thermal velocity of the molecules.

It has been surprisingly found that unsolubilized oil-soluble C18-C19 straight chain hydrocarbon in their crystalline form that have a heat of fusion that is higher than 100 J/g and a melting point between 30°-40°C, preferably between 30°-38°C. provide a cooling sensation when applied to the skin. While not wishing to be bound by any theories, it is believed that the relatively high heat of fusion facilitates the absorption of heat from the skin to aid in melting the solid ingredient when applied to the skin, thereby cooling the skin temperature. The melting point of the ingredient is substantially similar to human body temperature so that the ingredient only melts upon application to the skin.

It has been surprisingly found that having a heat of fusion that is higher than 100 J/g with an upper limit within a cosmetically acceptable range to avoid a negative affect on the human skin, and a melting point that is substantially similar to human body temperature, and that remain in their crystalline state in a formulation, cause a cooling sensation when applied to the skin. Because it is generally known in the art that ingredients are solubilized when incorporated into cosmetically suitable compositions and therefore lose their original structure, such ingredients therefore correspondingly lose their ability to provide a cooling sensation upon contact with the skin. The present invention surprisingly overcomes the problem by providing a composition containing a polymeric emulsion which supports the oil-soluble hydrophobic ingredients in their unsolubilized state, thereby surprisingly imparting a cooling sensation when the cosmetic composition is applied onto the skin. Such a composition provides a prolonged cooling sensation without the usual stinging sensation or strong odor.

Examples of suitable C18-C19 straight chain hydrocarbons n-Octadecane are n-Nonadecane and stearyl heptanoate. In the preferred embodiment, stearyl heptanoate is used in an amount of 8% to 70%, preferably from 15% to 60% and most preferably from 30% to 40%.

The cooling ingredients are incorporated in a polymeric emulsifier selected so that the emulsifier does not solubilize the cooling ingredients. It has been surprisingly found that cross-linked copolymers which can entrap oily components while physically anchoring such components within an emulsion, do not solubilize the cooling ingredients of the present invention. Therefore, suitable polymeric emulsifiers to be used in the present invention are emulsifiers that have a small lipophilic portion as well as a large lipophobic portion to carry out the anchoring and entrapping functions, respectively.

In a preferred embodiment, suitable polymeric emulsifiers include but are not limited to cross-linked copolymers of acrylic acid and C10-C30 alkyl acrylates. Most preferably, acrylates/C10-C30 alkyl acrylate crosspolymer is used (Pemulen® from Noveon, Inc. in Cleveland, Ohio).

The polymeric emulsifier is used in an amount of from 10 to 60%, preferably from 15 to 55% and most preferably from 30% to 40% by weight of the composition.

The composition further comprises a cosmetically acceptable vehicle that is suitable for topical application to skin, hair and/or nails.

The composition is in the form of an emulsion. Since the cooling ingredient must remain in an unsolubilized state, only water-soluble materials that don't have any emulsifying properties may be used in the composition. Ingredients which may be included in the composition include, for example, water-soluble moisturizing agents, water-soluble astringent agents, water-soluble film-forming materials, water-soluble sunscreens, water-soluble pigments or water-soluble proteins and/or fibers.

For purposes of the present invention, vehicles are preferably hydrophilic substances that can act as a carrier for the cooling ingredient. Hydrophilic carriers known to those skilled in the art may be used. The vehicle or vehicles can comprise from about 1 to about 99.9%, preferably from about 50 to about 99.5%, more preferably from about 70 to about 99.3% and most preferably from 80 to 90% by weight of the compositions.

The preferred embodiment of the present invention is in the form of an emulsion lotion for use in after-sun, after-shave and body moisturizing products.

The composition of the present invention may also contain additional water-soluble cosmetic ingredients that add to the aesthetics of performance of the present invention. For example, water-soluble pigments, water-soluble thickeners, lipophobic powders and non-oilbased fragrances may be used to increase the aesthetic appeal of the present invention.

The fragrance may be in an amount sufficient to make the composition more appealing to the consumer. Preferably, the fragrance is in the amount of from about 0.01 to about 10% by weight of the composition.

In addition to the elements described above, the skin care compositions of the present invention may include skin care actives. Actives, for purposes of the present invention, are defined as water-soluble skin or hair benefit agents other than emollients and other than ingredients that merely improve the physical characteristics of the composition. Examples of actives that may be useful include, but are not limited to, agents for the eradication of age spots, keratoses and wrinkles, analgesics, anesthetics, anti-acne agents, antibacterials, antiyeast agents, antifungal agents, antiviral agents, antidandruff agents, antidermatitis agents, antipruritic agents, antiemetics, antimotion sickness agents, anti-inflammatory agents, antihyperkeratolytic agents, anti-dry skin agents, antiperspirants, antipsoriatic agents, antiseborrheic agents, hair conditioners and hair treatment agents, antiaging agents, antiwrinkle agents, antiasthmatic agents and bronchodilators, sunscreen agents, antihistamine agents, skin lightening agents, depigmenting agents, vitamins, corticosteroids, self-tanning agents, hormones, and topical cardiovascular agents.

### METHODS FOR CAUSING COOLING SENSATION

The present inventive compositions are particularly useful for creating a cooling sensation on the skin. Specifically, the unsolubilized oil-soluble hydrophobic cooling ingredient of claim 1, surprisingly causes a cooling sensation upon topical application of the composition onto the skin.

The present composition is useful as an after-shave, after-sun, deodorant, anti-irritant or other skin care product. The preferred use of the present invention is as an after-sun or after-shave product to provide a soothing cooling sensation. The composition of the present invention may be applied on the skin directly after shaving in an amount as desired by the individual consumer.

The present invention may also be used for methods of regulating skin irritation or skin disorders with the addition of a water-soluble active, including but not limited to skin disorders such as dry skin, severe dry skin, xerosis, dandruff, keratoses, psoriasis, eczema, age spots, lentigines, melasmas, blemished skin, hyperpigmented skin, hyperkeratotic skin, inflammatory dermatoses and age-related skin changes. Such a method comprises administering or topically applying to the skin a safe and effective amount of the combination of the present invention, which further comprises the active component. The amounts of the components in the compositions will vary widely depending upon the level of skin aging already in existence in the subject (if such exists), the rate of further aging, and the level of regulation desired.

The regimen of application will depend on the ultimate intended use of the composition. For example, the composition may be applied on an as needed basis when a cooling sensation is desired, especially in an after-sun or after-shave product to cool the skin due to burning, irritation or even inflammation. Alternatively, a chronic application may be desired, particularly with a composition containing an active agent to regulate skin aging and to treat other skin maladies described above. A preferred amount of treating the skin is via topical application of a safe and effective amount of the novel composition provide a cooling sensation after sun exposure or shaving, described above.

It is suggested as an example that topical application range from about once per week to about 4 or 5 times daily, preferably from about 3 times a week to once daily. The compositions will comprise from about 0.01 to 90 %, preferably from about 1 to 20%, and most preferably from about 1 to 5 % of the active components. However, it should be noted that it is well within the purview of the skilled artisan, such as a dermatologist or other health care provider, to regulate pharmaceutical dosages according to patient needs, when an active is incorporated into the composition.

The following examples further illustrate the novel compositions and methods of the present invention, but the invention is not limited thereto.

### EXAMPLE 1

The following composition provides an example of a preferred embodiment incorporating the inventive composition in the form of an emulsion. The examples that follow are conducted utilizing the composition below.

| Trade Name | CTFA name | Percent |
|---|---|---|
| Deionized Water | Purified Water | 19.39 |
| Pemulen TR-1 (2% disp in Methylparaben) | Acrylates/C10-C30 Alkyl Acrylate Crosspolymer | 40.00 |
| Germall 115 | Imidazolidinyl Urea | 0.300 |
| Tegosoft SH | Stearyl Heptanoate | 40.00 |
| Caustic Soda 30% | Water/Sodium Hydroxide | 0.200 |
| FD&C Blue #1 (1% Aq. Sol) | Dye | 0.010 |
| Cilantro Fresca 475.251 EB | Fragrance | 0.100 |

### Example 2

The following experiment is conducted to test the cooling effect/cooling potential of stearyl heptanoate of the present invention in comparison with an ingredient that does not have a heat of fusion of above 100 J/g and a melting point between 30-40°C as is required by the present invention.

Four samples of the composition are prepared with 10%,20% and 40% of the stearyl heptanoate in three samples and 40% Marrix (C12-C15 Alkyl Furmurate) in a fourth sample. Each sample is first weighed and subsequently cooled to -10°C. A Calorimeter (Pyris 1 Differential Scanning Calorimeter from Perkin Elmer) is used to apply heat to each sample at one minute increments, 10°C per minute up to 50°C. The Calorimeter measures the heat of the sample at each increment. The resulting readings of heat are plotted against time for each sample. The Calorimeter measures the area under the peak on the plot to calculate the Heat of fusion (J/g) for each sample. The heat of fusion for the sample is the energy required to melt the sample from a solid form to a liquid form. The heat of fusion for the sample identifies the amount of energy the sample can absorb. Therefore, to correlate to the cooling on the skin, the higher the heat of fusion of a sample/material, the more heat the sample/material will absorb from the skin upon application to correspondingly create a cooling sensation on the skin.

**TABLE 1**

| Composition | (ΔH) Heat of fusion (J/g) |
|---|---|
| 10% Active | 18.6 |
| 20% Active | 37.7 |
| 40% Active | 74.5 |
| 40% Marrix | 41.5 |
| Active alone | 174.3 |

As seen in Table 1 above, the present inventive cooling ingredient, stearyl heptanoate, has a much higher heat of fusion (74.5) than Marrix, an ingredient that does not fall within the definition of the present invention. As seen in Table 1, the present inventive composition surprisingly causes a cooling sensation upon the skin on application. The active alone is shown to have a heat of fusion of above 100J/g, specifically 174.3 J/g.

## Claims

1. An emulsion composition for topical application to the skin comprising: at least one unsolubilized oil-soluble C18-C19 straight chain hydrocarbon having a heat of fusion above 100 J/g and a melting point between 30°C and 40°C; a polymeric emulsifier in which the hydrocarbon is not soluble, wherein the amount of the polymeric emulsifier is 10 to 60% by weight of the composition; and a cosmetically acceptable carrier that does not solubilize the cooling ingredient.

2. The composition of claim 1 wherein the polymeric emulsifier is selected from cross-linked copolymers of acrylic acid and C10-C30 alkyl acrylates.

3. The composition of claim 1 wherein the cooling ingredient is stearyl heptanoate.

4. A method of causing a cooling sensation on the skin comprising applying to the skin an emulsion composition according to claim 1.

5. The method of claim 4 wherein the polymeric emulsifier is selected from cross-linked copolymers of acrylic acid and C10-C30 alkyl acrylates.

6. The method of claim 4 wherein the cooling ingredient is stearyl heptanoate.

## Patentansprüche

1. Eine Emulsionszusammensetzung für die örtliche Anwendung auf der Haut bestehend aus:
- mindestens einem nicht löslich gemachten, öllöslichen, geradkettigen C₁₈-C₁₉ Kohlenwasserstoff mit einer Schmelzwärme oberhalb von 100 J/g und einem Schmelzpunkt zwischen 30°C und 40°C;
- einem polymerischen Emulgiermittel in dem der Kohlenwasserstoff nicht löslich ist, wobei die Menge des polymerischen Emulgiermittels 10 bis 60% des Gewichts der Zusammensetzung beträgt;
- und einem kosmetisch verträglichen Träger der nicht den kühlenden Bestandteil löst.

2. Die Zusammensetzung nach Anspruch 1, wobei das polymerische Emulgiermittel aus vernetzten Acrylsäurecopolymeren und C₁₀-C₃₀ Alkylacrylaten ausgewählt ist.

3. Die Zusammensetzung nach Anspruch 1, wobei der kühlende Bestandteil ein Stearylheptanoat ist.

4. Ein Verfahren um ein kühlendes Gefühl auf der Haut hervorzurufen, bestehend aus einer Emulsionszusammensetzung nach Anspruch 1, die auf die Haut aufgetragen wird.

5. Ein Verfahren nach Anspruch 4, wobei das polymerische Emulgiermittel aus vernetzten Acrylsäurecopolymeren und C₁₀-C₃₀ Alkylacrylaten ausgewählt ist.

6. Ein Verfahren nach Anspruch 4, wobei der kühlende Bestandteil ein Stearylheptanoat ist.

## Revendications

1. Une composition sous forme d'émulsion pour application topique sur la peau comprenant au moins un hydrocarbure à chaîne linéaire en C₁₈-C₁₉ soluble dans l'huile, non solubilisé, présentant une chaleur de fusion supérieure à 100J/g et un point de fusion compris entre 30°C et 40°C; comprenant un émulsifiant polymère dans lequel l'hydrocarbure n'est pas soluble, dans laquelle la quantité de l'émulsifiant polymère forme de 10 à 60% en poids de la composition et qui contient un support cosmétiquement acceptable ne solubilisant pas l'agent de refroidissement

2. La composition selon la revendication 1, dans laquelle l'émulsifiant polymère est choisi parmi les copolymères réticulés formés à partir d'acide acrylique et d'acrylates d'alkyle en C₁₀-C₃₀.

3. La composition selon la revendication 1, dans laquelle l'agent de refroidissement est l'heptanoate de stéaryle.

4. Un procédé pour produire une sensation de fraîcheur sur la peau comprenant l'application sur la peau d'une composition sous forme d'émulsion selon la revendication 1.

5. Un procédé selon la revendication 4, dans lequel l'émulsifiant polymère est choisi parmi les copolymères réticulés formés à partir d'acide acrylique et d'acrylates d'alkyle en C₁₀-C₃₀.

6. Le procédé selon la revendication 4, dans laquelle l'agent de refroidissement est l'heptanoate de stéaryle.
